# EUROPEAN PATENT SPECIFICATION

(11) **EP 2 398 922 B1**
(45) Date of publication and mention of the grant of the patent: **29.04.2015**
(21) Application number: 10793297.2
(22) Date of filing: 08.11.2010
(51) Int. Cl.: C12Q 1/68, G01N 33/573

(54) **RECQL4/RECQL4 VARIANT-P53 COMPLEX FOR ALTERED MITOCHONDRIAL FUNCTION IN ROTHMUND-THOMSON SYNDROME**
RECQL4/RECQL4-VARIANTEN-P53-KOMPLEX ZUR MODIFIKATION DER MITOCHONDRIENFUNKTION BEIM ROTHMUND-THOMSON-SYNDROM
COMPLEXE VARIANT RECQL4/RECQL4-P53 POUR ALTÉRER LA FONCTION MITOCHONDRIALE DANS LE SYNDROME DE ROTHMUND-THOMSON

(30) Priority: 10.11.2009 IN DE23212009
(43) Date of publication of application: 28.12.2011
(73) Proprietor: National Institute Of Immunology, New Delhi 110067 (IN)
(72) Inventor: SENGUPTA, Sagar, New Delhi 110067 (IN)
(74) Representative: Robertson, James Alexander
(86) International application number: PCT/IB2010/002834
(87) International publication number: WO 2011/058408

(56) References cited:
- EP-A1- 1 160 324
- MACRIS M A ET AL: "Biochemical characterization of the RECQ4 protein, mutated in Rothmund-Thomson syndrome", DNA REPAIR, ELSEVIER, AMSTERDAM, NL, vol. 5, no. 2, 3 February 2006 (2006-02-03), pages 172-180, XP026953641, ISSN: 1568-7864, DOI: 10.1038/nrm1546 [retrieved on 2006-01-13]
- SENGUPTA SAGAR ET AL: "Tumor suppressor p53 represses transcription of RECQ4 helicase.", ONCOGENE 3 MAR 2005 LNKD- PUBMED:15674334, vol. 24, no. 10, 3 March 2005 (2005-03-03) , pages 1738-1748, XP002631817, ISSN: 0950-9232, DOI: 10.1016/j.dnarep.2005.09.005
- LINDOR N M ET AL: "ROTHMUND-THOMSON SYNDROME DUE TO RCQ4 HELICASE MUTATIONS: REPORT AND CLINICAL AND MOLECULAR COMPARISONS WITH BLOOM SYNDROME AND WERNER SYNDROME", AMERICAN JOURNAL OF MEDICAL GENETICS, WILEY, NEW YORK,NY, US, vol. 90, no. 3, 31 January 2000 (2000-01-31), pages 223-228, XP001056302, ISSN: 0148-7299, DOI: DOI:10.1002/(SICI)1096-8628(20000131)90:3< 223::AID-AJMG7>3.0.CO;2-Z
- KITAO S ET AL: "Rothmund-Thomson Syndrome Responsible Gene, RECQL4: Genomic Structure and Products", GENOMICS, ACADEMIC PRESS, SAN DIEGO, US, vol. 61, no. 3, 1 November 1999 (1999-11-01), pages 268-276, XP004444765, ISSN: 0888-7543, DOI: DOI:10.1006/GENO.1999.5959
- NAVARRO CLAIRE L ET AL: "Molecular bases of progeroid syndromes", HUMAN MOLECULAR GENETICS, vol. 15, no. Sp. Iss. 2, October 2006 (2006-10), pages R151-R161, XP002631818, DOI: 10.1093/hmg/ddl214
- LARIZZA L ET AL: "Rothmund-Thomson syndrome and RECQL4 defect: Splitting and lumping", CANCER LETTERS, NEW YORK, NY, US, vol. 232, no. 1, 28 January 2006 (2006-01-28), pages 107-120, XP025021479, ISSN: 0304-3835, DOI: DOI:10.1016/J.CANLET.2005.07.042 [retrieved on 2006-01-28]
- XU XIAOHUA ET AL: "MCM10 mediates RECQ4 association with MCM2-7 helicase complex during DNA replication", EMBO (EUROPEAN MOLECULAR BIOLOGY ORGANIZATION) JOURNAL, vol. 28, no. 19, October 2009 (2009-10), pages 3005-3014, XP002631819, ISSN: 0261-4189
- SENGUPTA SAGAR ET AL: "p53: traffic cop at the crossroads of DNA repair and recombination.", NATURE REVIEWS. MOLECULAR CELL BIOLOGY JAN 2005 LNKD- PUBMED:15688066, vol. 6, no. 1, January 2005 (2005-01), pages 44-55, XP002631820, ISSN: 1471-0072

### SEQUENCE LISTING.

<110> National Institute of Immunology
<120> RECQL4/RECQL4 variant -p53 Complex for Altered Mitochondrial Function in Rothmund-Thomson Syndrome
<130> IP12102
<140> 2321/DEL/2009 <141> 2009-11-10
<160> 3
<170> PatentIn version 3.5
<210> 1
   <211> 459
   <212> PRT
   <213> Human
<400> 1
   Met Glu Arg Leu Arg Asp Val Arg Glu Arg Leu Gln Ala Trp Glu Arg
   1               5                   10                  15
   Ala Phe Arg Arg Gln Arg Gly Arg Arg Pro Ser Gln Asp Asp Val Glu
               20                  25                  30
   Ala Ala Pro Glu Glu Thr Arg Ala Leu Tyr Arg Glu Tyr Arg Thr Leu
           35                  40                  45
   Lys Arg Thr Thr Gly Gln Ala Gly Gly Gly Leu Arg Ser Ser Glu Ser
       50                  55                  60
   Leu Pro Ala Ala Ala Glu Glu Ala Pro Glu Pro Arg Cys Trp Gly Pro
   65                  70                  75                  80
   His Leu Asn Arg Ala Ala Thr Lys Ser Pro Gln Pro Thr Pro Gly Arg
                   85                  90                  95
   Ser Arg Gln Gly Ser Val Pro Asp Tyr Gly Gln Arg Leu Lys Ala Asn
               100                 105                 110
   Leu Lys Gly Thr Leu Gln Ala Gly Pro Ala Leu Gly Arg Arg Pro Trp
           115                 120                 125
   Pro Leu Gly Arg Ala Ser Ser Lys Ala Ser Thr Pro Lys Pro Pro Gly
       130                 135                 140
   Thr Gly Pro Val Pro Ser Phe Ala Glu Lys Val Ser Asp Glu Pro Pro
   145                 150                 155                 160
   Gln Leu Pro Glu Pro Gln Pro Arg Pro Gly Arg Leu Gln His Leu Gln
                   165                 170                 175
   Ala Ser Leu Ser Gln Arg Leu Gly Ser Leu Asp Pro Gly Trp Leu Gln
               180                 185                 190
   Arg Cys His Ser Glu Val Pro Asp Phe Leu Gly Ala Pro Lys Ala Cys
   195 200 205
   Arg Pro Asp Leu Gly Ser Glu Glu Ser Gln Leu Leu Ile Pro Gly Glu
   210 215 220
   Ser Ala Val Leu Gly Pro Gly Ala Gly Ser Gln Gly Pro Glu Ala Ser
   225 230 235 240
   Ala Phe Gln Glu Val Ser Ile Arg Val Gly Ser Pro Gln Pro Ser Ser
   245 250 255
   Ser Gly Gly Glu Lys Arg Arg Trp Asn Glu Glu Pro Trp Glu Ser Pro
   260 265 270
   Ala Gln Val Gln Gln Glu Ser Ser Gln Ala Gly Pro Pro Ser Glu Gly
   275 280 285
   Ala Gly Ala Val Ala Val Glu Glu Asp Pro Pro Gly Glu Pro Val Gln
   290 295 300
   Ala Gln Pro Pro Gln Pro Cys Ser Ser Pro Ser Asn Pro Arg Tyr His
   305 310 315 320
   Gly Leu Ser Pro Ser Ser Gln Ala Arg Ala Gly Lys Ala Glu Gly Thr
   325 330 335
   Ala Pro Leu His Ile Phe Pro Arg Leu Ala Arg His Asp Arg Gly Asn
   340 345 350
   Tyr Val Arg Leu Asn Met Lys Gln Lys His Tyr Val Arg Gly Arg Ala
   355 360 365
   Leu Arg Ser Arg Leu Leu Arg Lys Gln Ala Trp Lys Gln Lys Trp Arg
   370 375 380
   Lys Lys Gly Glu Cys Phe Gly Gly Gly Gly Ala Thr Val Thr Thr Lys
   385 390 395 400
   Glu Ser Cys Phe Leu Asn Glu Gln Phe Asp His Trp Ala Ala Gln Cys
   405 410 415
   Pro Arg Pro Ala Ser Glu Glu Asp Thr Asp Ala Val Gly Pro Glu Pro
   420 425 430
   Leu Val Pro Ser Pro Gln Pro Val Pro Glu Val Pro Ser Leu Asp Pro
   435 440 445
   Thr Val Leu Pro Leu Tyr Ser Leu Gly Pro Ser
   450 455
<210> 2
   <211> 409
   <212> PRT
   <213> Human
<400> 2
   Gly Gln Leu Ala Glu Thr Pro Ala Glu Val Phe Gln Ala Leu Glu Gln
   1               5                   10                  15
   Leu Gly His Gln Ala Phe Arg Pro Gly Gln Glu Arg Ala Val Met Arg
               20                  25                  30
   Ile Leu Ser Gly Ile Ser Thr Leu Leu Val Leu Pro Thr Gly Ala Gly
           35                  40                  45
   Lys Ser Leu Cys Tyr Gln Leu Pro Ala Leu Leu Tyr Ser Arg Arg Ser
       50                  55                  60
   Pro Cys Leu Thr Leu Val Val Ser Pro Leu Leu Ser Leu Met Asp Asp
   65                  70                  75                  80
   Gln Val Ser Gly Leu Pro Pro Cys Leu Lys Ala Ala Cys Ile His Ser
                   85                  90                  95
   Gly Met Thr Arg Lys Gln Arg Glu Ser Val Leu Gln Lys Ile Arg Ala
               100                 105                 110
   Ala Gln Val His Val Leu Met Leu Thr Pro Glu Ala Leu Val Gly Ala
           115                 120                 125
   Gly Gly Leu Pro Pro Ala Ala Gln Leu Pro Pro Val Ala Phe Ala Cys
       130                 135                 140
   Ile Asp Glu Ala His Cys Leu Ser Gln Trp Ser His Asn Phe Arg Pro
   145                 150                 155                 160
   Cys Tyr Leu Arg Val Cys Lys Val Leu Arg Glu Arg Met Gly Val His
                   165                 170                 175
   Cys Phe Leu Gly Leu Thr Ala Thr Ala Thr Arg Arg Thr Ala Ser Asp
               180                 185                 190
   Val Ala Gln His Leu Ala Val Ala Glu Glu Pro Asp Leu His Gly Pro
           195                 200                 205
   Ala Pro Val Pro Thr Asn Leu His Leu Ser Val Ser Met Asp Arg Asp
       210                 215                 220
   Thr Asp Gln Ala Leu Leu Thr Leu Leu Gln Gly Lys Arg Phe Gln Asn
   225                 230                 235                 240
   Leu Asp Ser Ile Ile Ile Tyr Cys Asn Arg Arg Glu Asp Thr Glu Arg
                   245                 250                 255
   Ile Ala Ala Leu Leu Arg Thr Cys Leu His Ala Ala Trp Val Pro Gly
               260                 265                 270
   Ser Gly Gly Arg Ala Pro Lys Thr Thr Ala Glu Ala Tyr His Ala Gly
   275 280 285
   Met Cys Ser Arg Glu Arg Arg Arg Val Gln Arg Ala Phe Met Gln Gly
   290 295 300
   Gln Leu Arg Val Val Val Ala Thr Val Ala Phe Gly Met Gly Leu Asp
   305 310 315 320
   Arg Pro Asp Val Arg Ala Val Leu His Leu Gly Leu Pro Pro Ser Phe
   325 330 335
   Glu Ser Tyr Val Gln Ala Val Gly Arg Ala Gly Arg Asp Gly Gln Pro
   340 345 350
   Ala His Cys His Leu Phe Leu Gln Pro Gln Gly Glu Asp Leu Arg Glu
   355 360 365
   Leu Arg Arg His Val His Ala Asp Ser Thr Asp Phe Leu Ala Val Lys
   370 375 380
   Arg Leu Val Gln Arg Val Phe Pro Ala Cys Thr Cys Thr Cys Thr Arg
   385 390 395 400
   Pro Pro Ser Glu Gln Glu Gly Ala Val
   405
<210> 3
   <211> 340
   <212> PRT
   <213> Human
<400> 3
   Gly Gly Glu Arg Pro Val Pro Lys Tyr Pro Pro Gln Glu Ala Glu Gln
   1               5                   10                  15
   Leu Ser His Gln Ala Ala Pro Gly Pro Arg Arg Val Cys Met Gly His
               20                  25                  30
   Glu Arg Ala Leu Pro Ile Gln Leu Thr Val Gln Ala Leu Asp Met Pro
           35                  40                  45
   Glu Glu Ala Ile Glu Thr Leu Leu Cys Tyr Leu Glu Leu His Pro His
       50                  55                  60
   His Trp Leu Glu Leu Leu Ala Thr Thr Tyr Thr His Cys Arg Leu Asn
   65                  70                  75                  80
   Cys Pro Gly Gly Pro Ala Gln Leu Gln Ala Leu Ala His Arg Cys Pro
                   85                  90                  95
   Pro Leu Ala Val Cys Leu Ala Gln Gln Leu Pro Glu Asp Pro Gly Gln
               100                 105                 110
   Gly Ser Ser Ser Val Glu Phe Asp Met Val Lys Leu Val Asp Ser Met
   115 120 125
   Gly Trp Glu Leu Ala Ser Val Arg Arg Ala Leu Cys Gln Leu Gln Trp
   130 135 140
   Asp His Glu Pro Arg Thr Gly Val Arg Arg Gly Thr Gly Val Leu Val
   145 150 155 160
   Glu Phe Ser Glu Leu Ala Phe His Leu Arg Ser Pro Gly Asp Leu Thr
   165 170 175
   Ala Glu Glu Lys Asp Gln Ile Cys Asp Phe Leu Tyr Gly Arg Val Gln
   180 185 190
   Ala Arg Glu Arg Gln Ala Leu Ala Arg Leu Arg Arg Thr Phe Gln Ala
   195 200 205
   Phe His Ser Val Ala Phe Pro Ser Cys Gly Pro Cys Leu Glu Gln Gln
   210 215 220
   Asp Glu Glu Arg Ser Thr Arg Leu Lys Asp Leu Leu Gly Arg Tyr Phe
   225 230 235 240
   Glu Glu Glu Glu Gly Gln Glu Pro Gly Gly Met Glu Asp Ala Gln Gly
   245 250 255
   Pro Glu Pro Gly Gln Ala Arg Leu Gln Asp Trp Glu Asp Gln Val Arg
   260 265 270
   Cys Asp Ile Arg Gln Phe Leu Ser Leu Arg Pro Glu Glu Lys Phe Ser
   275 280 285
   Ser Arg Ala Val Ala Arg Ile Phe His Gly Ile Gly Ser Pro Cys Tyr
   290 295 300
   Pro Ala Gln Val Tyr Gly Gln Asp Arg Arg Phe Trp Arg Lys Tyr Leu
   305 310 315 320
   His Leu Ser Phe His Ala Leu Val Gly Leu Ala Thr Glu Glu Leu Leu
   325 330 335
   Gln Val Ala Arg
   340

## Description

### FIELD OF INVENTION:

The present invention is in the field of biotechnology.

### BACKGROUND AND PRIOR ART:

Maintenance of genomic integrity is critical as its instability is implicated in development and progression of cancer and in the aging process. One such class of enzymes involved in maintaining genomic integrity is the RecQ helicases which are encoded in human by the BLM, WRN and RECQL4 genes. Mutations in these genes result in autosomal recessive disorders such as Bloom syndrome (BS), Werner's Syndrome (WS) and Rothmund-Thomson Syndrome (RTS). RECQL4 gene encodes a protein of 1208 amino acids with a centrally conserved helicase domain. Sequences in the N- and C-terminal regions of RECQL4 protein do not have any similarity to other members of the RecQ helicases, indicating their importance in mediating specific functions. RECQL4, the protein was shown to have both nuclear and cytoplasmic localization (Petkovic et al., 2005; Werner et al., 2006; Yin et al., 2004). p300-mediated acetylation of RECQL4 may regulate the trafficking between nucleus and cytoplasm (Dietschy et al., 2009). Interaction of tumor suppressor gene p53 physically and functionally with members of the RecQ family of DNA helicases has been established. p53 can bind to both BLM and WRN and attenuate their activity to unwind Holliday Junctions (HJ) *in vitro* (Yang et al., 2002). Endogenous BLM and p53 bind in presence of DNA damage (Sengupta et al., 2003). Inactivation of p53 in BS cells led to a significant increase in the frequency of sister chromatid exchange (SCE), compared to BS alone, indicating that p53 and BLM cooperatively affect homologous recombination (HR). p53 can repress RECQL4 transcription by regulating the promoter occupancy of both itself and SP 1 in concert with histone deacetylase (HDAC1) (Sengupta et al., 2005).

EP 0999273 discloses the human gene RECQ4 and the proteins encoded by it and the variants of it. Also disclosed are antibodies to the RECQL4 protein, kits for diagnosing disease associated with RECQL4, transgenic and knockout animals of the RECQL4 gene and probes to the RECQL4 gene.

WO02/068672 is drawn to antisense compounds, compositions and methods for modulating nuclear RECQL4 expression, particularly with oligonucleotides hybridizable with nucleic acids encoding RECQL4.

WO2006/109091 discloses the identification of RECQL4 deficient condition and a method of treating a RECQL4 deficient cancer condition in an individual with the administration of DNA replication inhibitor to the individual.

Other publications include the following:
MACRIS M A ET AL., DNA REPAIR, ELSEVIER, AMSTERDAM, NL, vol. 5, no. 2, 3 February 2006 (2006-02-03), pages 172-180
SENGUPTA SAGAR ET AL., ONCOGENE 3 MAR 2005, vol. 24, no. 10, pages 1738-1748
LINDOR N M ET AL., AMERICAN JOURNAL OF MEDICAL GENETICS, WILEY, NEW YORK,NY, US, vol. 90, no. 3, 31 January 2000, pages 223-228
EP1160324
KITAO S ET AL., GENOMICS, ACADEMIC PRESS, SAN DIEGO, US, vol. 61, no. 3, 1 november 1999, pages 268-276
NAVARRO CLAIRE L ET AL., HUMAN MOLECULAR GENETICS, vol. 15, no. Sp. Iss. 2, October 2006, pages R151-R161
LARIZZA L ET AL., CANCER LETTERS, NEW YORK, NY, US, vol. 232, no. 1, 28 January 2006, pages 107-120
XU XIAOHUA ET AL., EMBO (EUROPEAN MOLECULAR BIOLOGY ORGANIZATION) JOURNAL, vol. 28, no. 19, October 2009, pages 3005-3014
SENGUPTA SAGAR ET AL., NATURE REVIEWS. MOLECULAR CELL BIOLOGY, vol. 6, no. 1, January 2005, pages 44-55

Studies have shown that cells exhibiting RTS condition unlike normal human fibroblasts, contain high levels of persistent reactive oxygen species (ROS) after DNA damage. It may be an indication of the mitochondrial dysfunction where there is disruption in the free radical scavenging enzymes, which in turn result in the cascade of reactions -from premature aging to neoplastic conversion of the normal cells into their cancerous counterpart. There are no conclusive studies on the functional interaction of RECQL4 and p53, although nuclear and cytoplasmic localization of both had been established.

The present invention thus aims:
Disclosing the functional interaction of the p53 with RECQL4.
Identification of RECQL4/ RECQL4 variant and p53 complex in the mitochondrial nucleoids.
Identification of mitochondrial localization signal (MLS) at the N-terminus of RECQL4 that binds to Tom 40 receptor complex and transport RECQL4/RECQL4 variant-p53 complex into the mitochondria.
Measurement of intracellular ROS and mitochondrial DNA (mtDNA) mutations as a diagnostic tool for RTS.
Treatment with ascorbic acid for scavenging ROS as a treatment for RTS.

### OBJECT OF THE INVENTION:

According to the present invention there is provided a method of identification of functional RECQL4/RECQL4 variant -p53 complex and of mitochondrial mutations in Rothmund-Thomson Syndrome (RTS) as defined in the appended independent claim 1. Further preferable features are defined in the appended dependent claims.

### BRIEF DESCRIPTION OF FIGURES AND TABLES:

Figure 1 (A-B) depicts *in vitro* GST pull down assays to determine RECQL4-p53 interaction.
Figure 2 (A-G) illustrates the interaction of various mutant RECQL4 fragments with p53:
Figure 3 (A-B) illustrates the validation of RECQL4-p53 interaction using Flag-tagged RECQL4 and GFP tagged full-length p53.
Figure 4 (A-G) are the confocal photomicrographs of the co localization of RECQL4 with p53, BrdU and other mitochondrial proteins.
Figure 5 (A-G) depicts the involvement of Tom20 receptor (a member of the Tom40 receptor complex) in the transport of p53-RECQL4 into the mitochondria.
Figure 6 (A-D) depicts various models for identification of mitochondrial localisation signal in RECQL4.

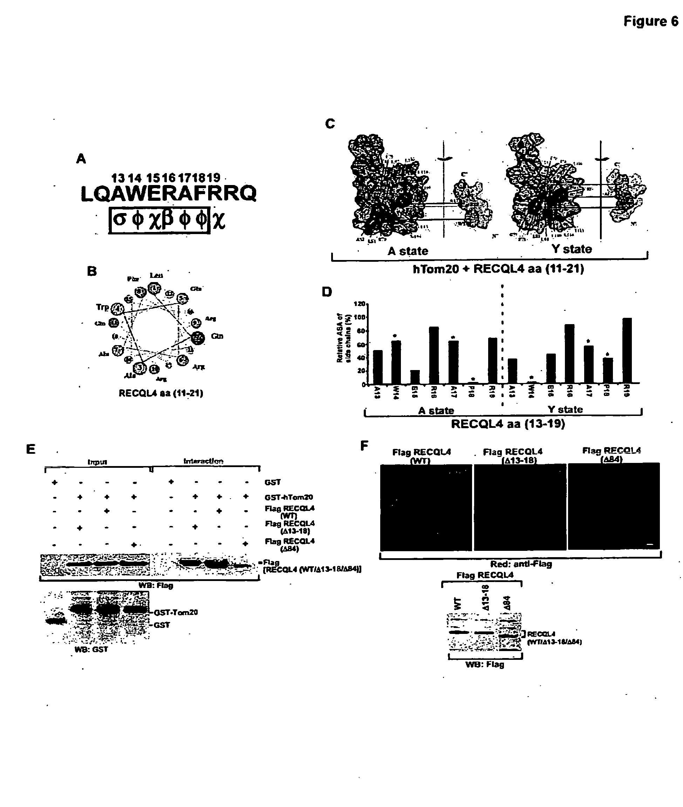

Figure 6 (E-F) illustrates the validation of the mitochondrial localisation signal in RECQL4 by studying the relative binding of the wildtype and mutant helicase with Tom20

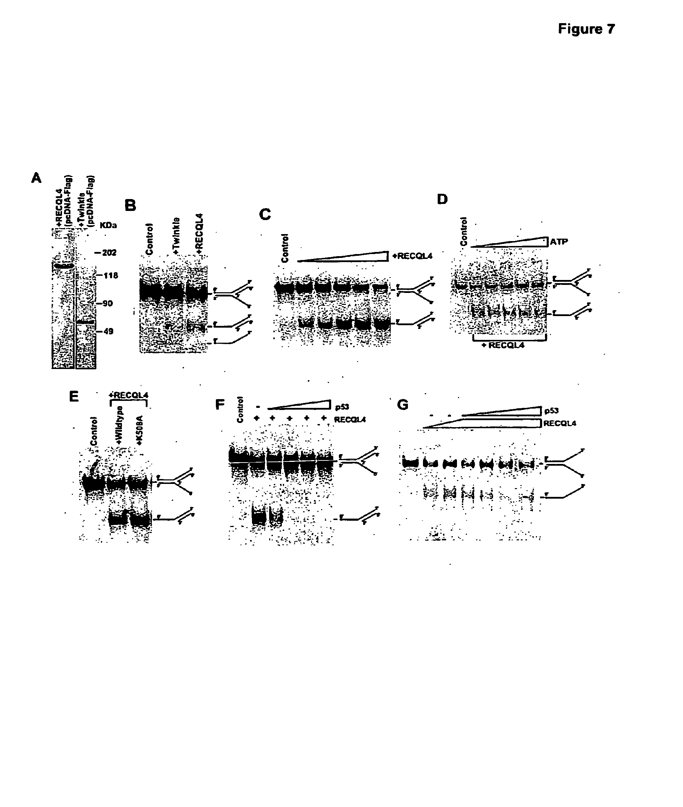

Figure 7 illustrates the helicase function of RECQL4 on different substrates a nd how p53 regulates that function.

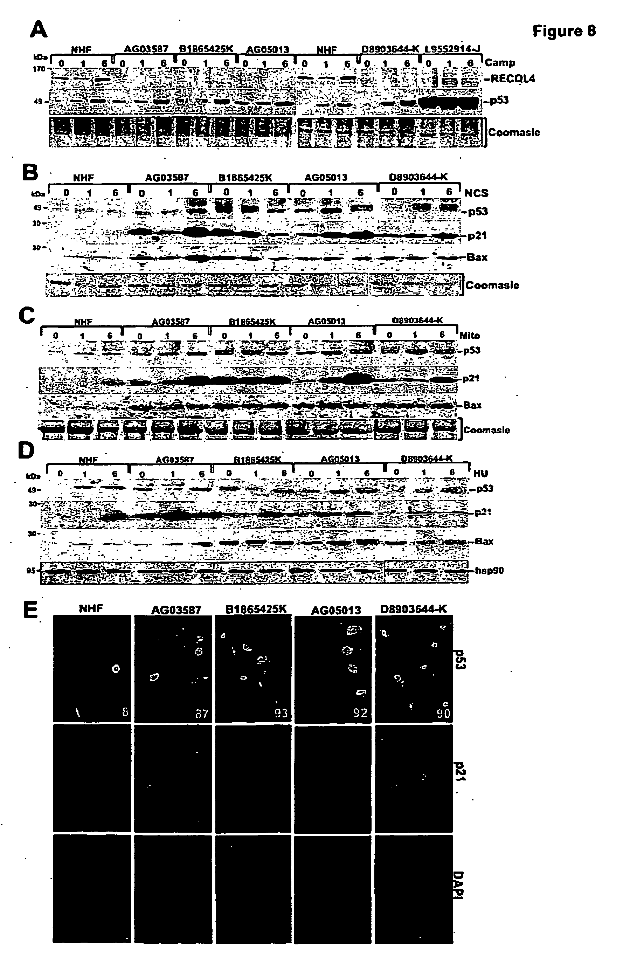

Figure 8 demonstrates how p53 and its down stream target genes (Bax and p21) are activated in absence of RECQL4 in RTS patients.

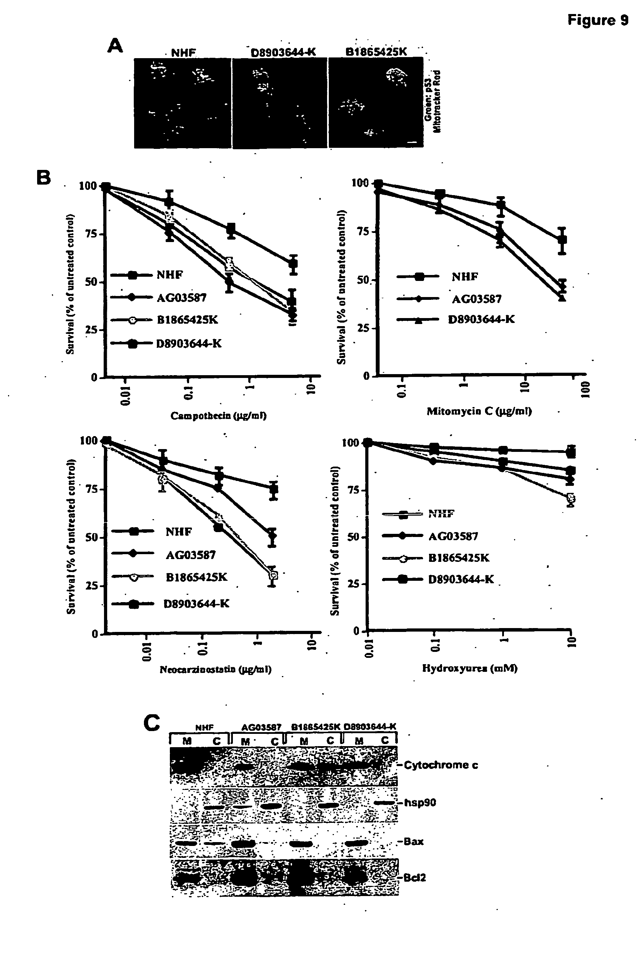

Figure 9A illustrates the constitutive nuclear localization of p53 due absence of RECQL4 in RTS patients.
Figure 9B shows how the viability of RTS patient cells are decreased when exposed to different types of DNA damage.
Figure 9C determines the mechanism leadingto the lack of spontaneous apoptosis in RTS patient cells.

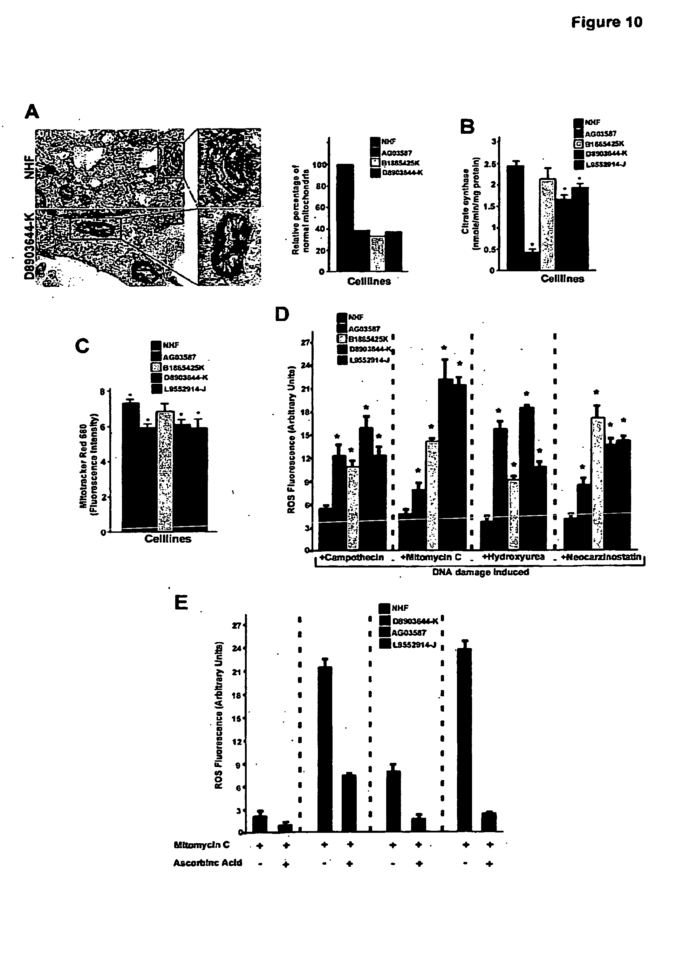

Figure 10A illustrates the altered mitochondrial ultrastructure in RTS patient cells.
Figure 10 (B-D) illustrates the change in the mitochondrial mass and level of reactive oxygen species in RTS patient cells after exposure to DNA damage.
Figure 10E demonstrates that treatment of RTS patient cells with ascorbic acid post exposure to mitomycin C decreases the load of reactive oxygen species.

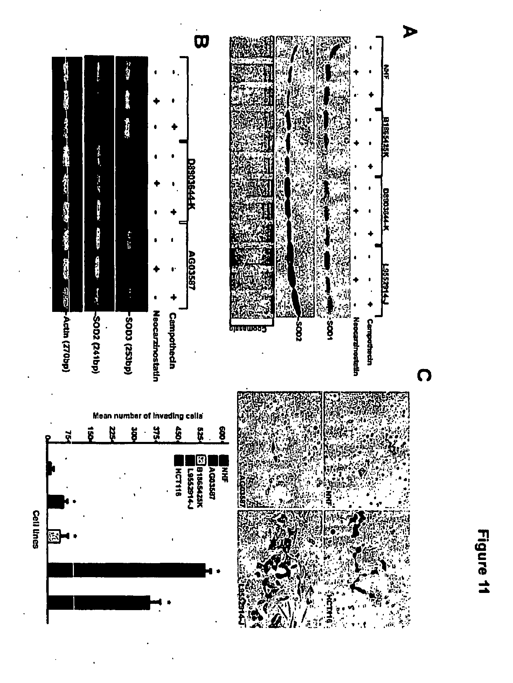

Figure 11 (A, B) illustrates the change in the levels of SOD1 and SOD2 in RTS patient cells relative to NHF.
Figure 11C illustrates the increase in the metastatic potential of the RTS patient cells.
Table 1 summarizes of prediction for RECQL4 for mitochondrial localization signal by different web based tools.
Table 2 showing the pair potential score for the interaction between Tom20 receptor structure and the predicted mitochondrial localization sequence in RECQL4
Table 3 lists the mutations detected in the mitochondrial DNA of RECQLA fibroblasts.

### DETAILED DESCRIPTION OF THE INVENTION:

Accordingly the present invention thus discloses the functional interaction of the p53 with RECQL4/RECQL4 variant. The present invention further discloses co localization of RECQL4/ RECQL4 variant and p53 complex in the mitochondrial nucleoids.

The invention further identifies MLS at the N-terminus of RECQL4 that binds to Tom 20 receptor complex and transport RECQL4/RECQL4 variant-p53 complex into the mitochondria.

Further the invention discloses measurement of intracellular ROS and identification of mtDNA mutations as a diagnostic tool for RTS.

Further the invention discloses that the treatment with ascorbic acid for scavenging ROS and as treatment for RTS.

Accordingly, the present invention provides a method of identification of functional RECQL4/RECQL4 variant -p53 complex wherein the said method comprising the steps of:
a) interaction of p53 and RECQL4/RECQL4 variant
b) import of RECQL4/RECQL4 variant and p53 complex through Tom 40 receptor complex.
c) import of RECQL4/RECQL4 variant and p53 complex through Tom 40 receptor complex.via the MLS of RECQL4.
d) the localization of the p53-RECQL4/RECQL4 variant complex in the mitochondrial nucleoids.

Further the invention discloses the interaction of RECQL4 and p53 resulting in masking of the nuclear localization signal of both RECQL4 and p53.

The functional MLS of RECQU is located at the N-terminal region, of RECQL4. The said MLS of RECQL4 is located between the 13th and 18th peptide of the N-terminal region of RECQLA.

The invention further discloses the lack of RECQL4 in RTS patients lead to deregulated mitochondrial functions.

Also, the invention discloses increased mtDNA mutations and enhanced metastatis of RTS fibroblasts with the loss of both RECQL4 and p53.

In another embodiment, the invention discloses a lentivirally expressed mitochondrially targeted RECQL4/RECQL4 variants which has the minimal interacting region for p53 and the helicase domain and can be used a vehicle to correct mitochondrial dysfunction. The "rescued" patient cells are then scored for the mitochondrial functions.

### EXAMPLES:

The following examples are for the purpose of illustration of the invention and are not intended in any way to limit the scope of the invention.

### EXAMPLE 1:

### Interaction between p53 and RECQL4 lead to the masking of their Nuclear Localization Signal

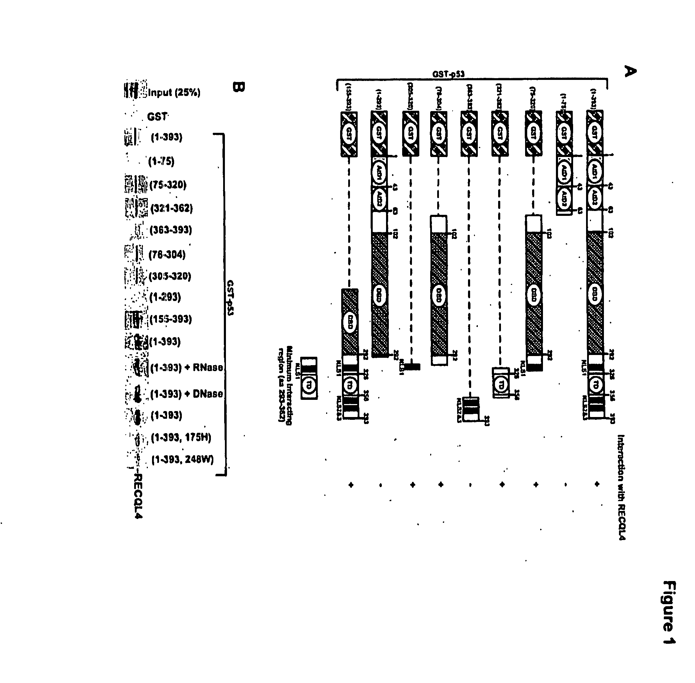

To determine RECQL4 interaction with p53 an *in vitro* GST pull-down assays with S35 labeled RECQL4 and the different deletion fragment of the tumor suppressor (Figure 1A, 1B) was carried out. It was found that the minimal region of p53 that interacts with RECQL4 encompasses the amino acids 293-362. This region contains the major Nuclear Localization Signal (NLS1) of p53 along with the tetramerization domain (TD). p53-RECQL4 interaction is independent of nucleic acids. Two tumor derived mutants of p53 (175H, 248W), also show interactions with RECQL4, albeit to a reduced level compared to the wild-type tumor suppressor.

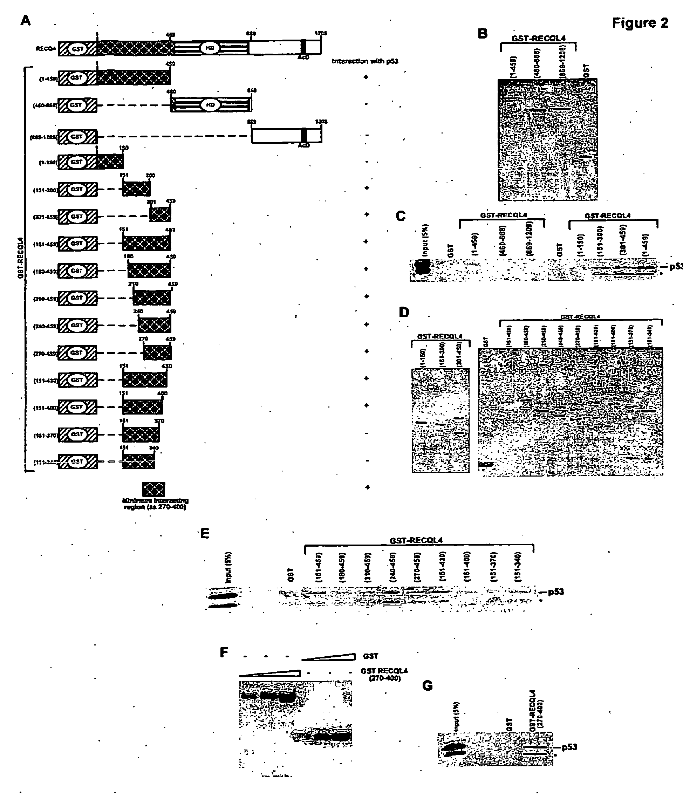

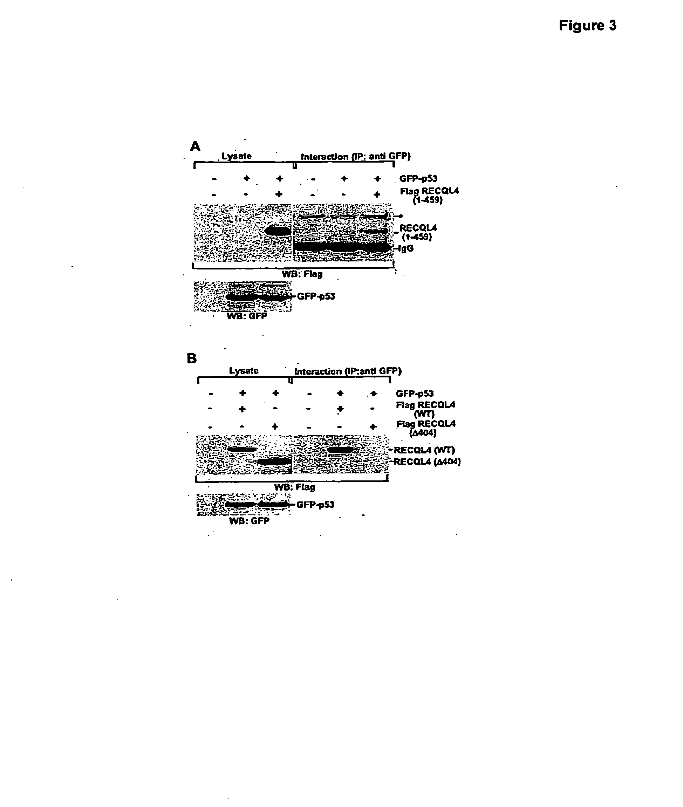

To determine the region of RECQL4, interaction with p53 three mutant protein fragments of RECQL4: 1-459, 460-868, 869-1208 (SEQ ID 1, 2 and 3) were generated and purified. The N-terminal 459 amino acids of RECQL4 interacted with p53 (Figure 2A, 2B, 2C). Subsequently, N- and C-terminal mutants were generated within the first 459 amino acids, purified and GST pull down assays with S35 labeled p53 were performed and a minimal stretch of 130 amino acids (270-400) of RECQL4 interacted with p53 (Figure 2D, 2E). The interaction with p53 was verified either with a GST-tagged RECQL4 (270-400) fragment *in vitro* (Figure 2F, 2F) or by using a Flag- tagged RECQL4 (1-459) fragment *in vivo* (Figure 3A). Further it was found that full-length RECQL4 but not the N-terminal 404 amino acid truncated variant interacted with GFP tagged full-length p53, thereby validating the region of interaction in the context of full length proteins (Figure 3B).

### EXAMPLE 2:

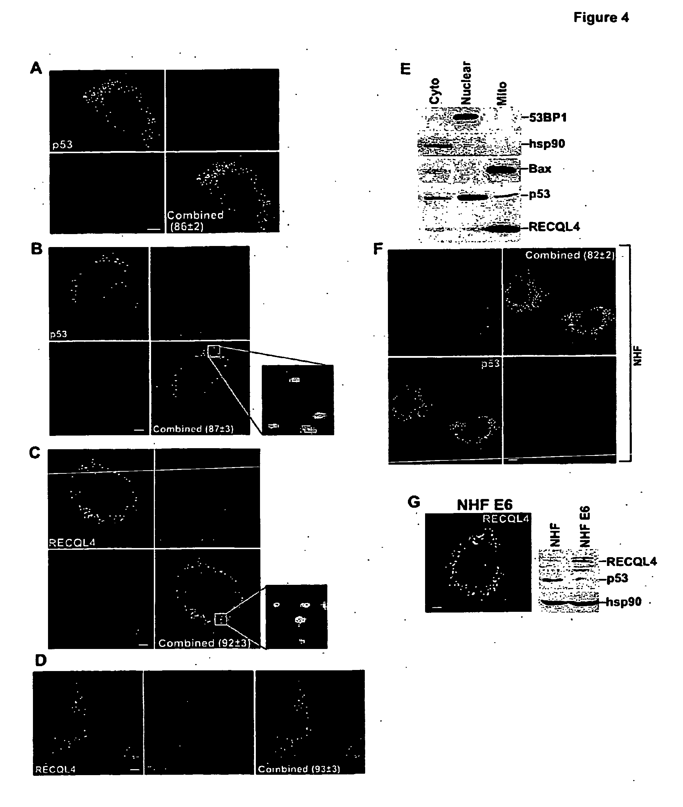

RECQL4 and p53 colocalize in mitochondria:
To determine the subcellular distribution of p53 and RECQL4/RECQL4 variant, we stained asynchronously grown NHF with p53 and RECQL4 antibodies and imaging with confocal microscopy was carried out. For confocal microscopy, the slides were analyzed on a Zeiss 510 Meta system with 63x/1.4 oil immersion or 40x10.95 Con objective. The laser lines used were Argon 458/477/488/514 nm (for FITC), DPSS 561 nm (for Texas Red and Mitotracker dyes) and a Chameleon Ultra autotunable femtosecond laser with a tuning range 690-1050 nm (for DAPI). LSM5 software was used for image acquisition. p53 and RECQL4 colocalized to a high degree in the cytoplasm as punctate staining (Figure 4A) and also found that both p53 and RECQL4 colocalized extensively with mitochondrial marker, cytochrome c and Mitotracker Red (Figure 4B, 4C) and mitochondrial helicase Twinkle (Figure 4D). The mitochondrial localization of RECQL4 and p53 was further demonstrated by cell fractionation experiment (Figure 4E). The integrity of the cell fractionation was tested by specific markers. Substantial amount of both p53 and RECQL4 were found to be present in nucleus and mitochondria. Since nucleoids contain mtDNA, we performed immuno-colocalization of RECQL4 and p53 with BrdU or Ethidium Bromide (EtBr) according to published protocols (Davis and Clayton 1996; Spelbrink et al. 2001). Both p53 and/or RECQL4 colocalized with BrdU (Figure 4F). Using NHF E6 cells the localization of RECQL4 with mtDNA was found to be independent of p53 (Figure 4G).

### EXAMPLE 3:

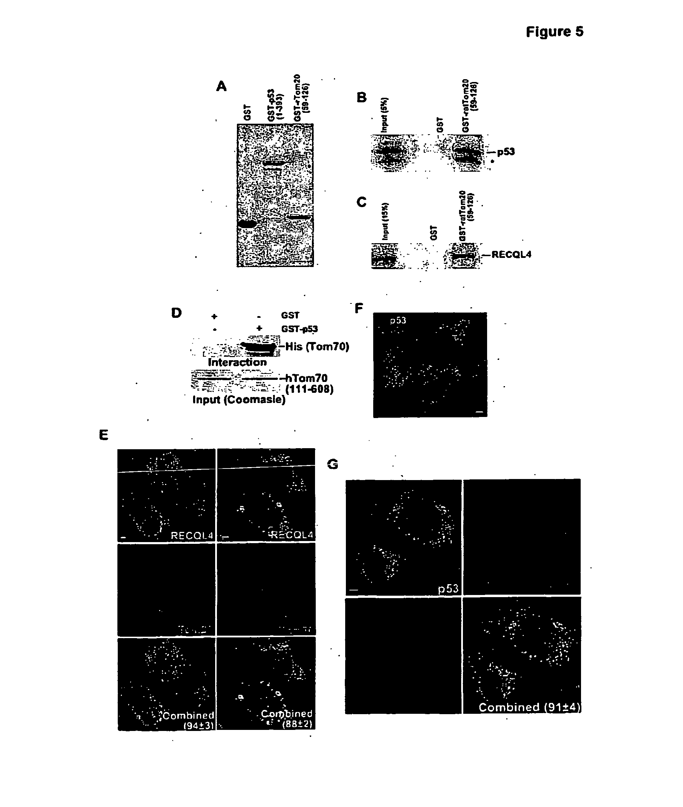

The MLS of RECQL4 was determined in the N-terminal region of the helicase: Since both p53 and RECQL4 were localized in the mitochondria (Figure 4) the interaction with mitochondrial receptor proteins was studied. Using S35-labeled RECQL4 and p53 we found that both the proteins interacted *in vitro* with cytosolic part of the Tom20 receptor (Figure 5A, 5B, 5C). p53 also interacted with the cytosolic part of Tom70 (Figure 5D). Both p53 and RECQL4 colocalized extensively with Tom20 and Tom70 (Figure 5E, 5F, 5G). Using four web based tools Target P, PSORT, MitoProt and iPSORT it was found that mitochondrial localization was predicted with high reliability and probability for RECQL4 especially the N-terminal domain (Table 1). We reasoned that the putative MLS of RECQL4 should be recognized by Tom20. A systematic analysis with the disulfide-tethering peptide library had been carried out to reveal the amino acid residues of the substrates involved in the interaction with Tom20 (Obita et al., 2003). The deduced motif was "σΦχβΦΦχ" where φ, Hydrophobic; σ, hydrophilic; β, basic; χ any amino acid. We searched the N-terminal 100 amino acids of RECQL4 using the above motif. Interestingly, one sequence stretch '13AWERAF18" which matched with the defined motif (Figure 6A) was found. The deduced pre-sequence was modeled onto the available solution structure [10M2.pdbj and the A state and Y state crystal structures of the rat Tom20 receptor [2VIT.pdb, 2VIS.pdbj (Saitoh et al., 2007) and binding affinity of this pre-sequence peptide with the Tom20 receptors was evaluated using a residue based statistical pair potential, namely the BT matrix (Betancourt and Thirumalai, 1999). Binding energies based on pair potentials gives us a score to distinguish between probable mitochondrial pre-sequence and non binders (Table 2), where we have compared the scores for the RECQL4 pre-sequence with the aldehyde dehydrogenase pre-sequence and a NLS in DNA helicase enzyme. The helical wheel plot also revealed that hydrophobic amino acids were concentrated on one side of the helix (which presumably faced the cytosol) while the hydrophilic amino acids were on the other side (which could bind to Tom20 receptor) (Figure 6B), indicating that the predicted pre sequence is the most probable MLS. To have a structural overview of RECQLA-Tom20 interaction, all atom models of the predicted pre-sequence in RECQL4 were built in complex with Tom20 using SCWRL (Canutescu et al., 2003). The models generated by SCWRL were further energy minimized using the discover module of Insight II. Co-crystal structure studies have revealed that Tom20 in solution can bind to atleast two bound states of the presequence (Saitoh et al., 2007). The two states of the presequence (A state and Y state) were obtained by the usage of different linker designs. The open-book representation of minimized structures for the Tom20 receptor-RECQL4 presequence for both states 10 revealed the importance of Trp 14, Ala 17 and Phe 18 of RECQL4 in the binding (Figure 6C). These residues form a hydrophobic surface of the amphiphilic ct-helix that interacts with the hydrophobic patch in the Tom20 groove. Two out of the three hydrophobic residues of the pre-sequence were tightly buried in the two different states of Tom20 receptor. This was further correlated by the relative surface accessibility of the side chain atoms of these hydrophobic residues in the modeled complexes of the A state and the Y state crystal structure of Tom20 receptor (Figure 6D). Overall the *in silico* experiments predicted the presence of a mitochondrial localization sequence in the extreme N-terminal region of RECQL4, which could bind to mitochondriale receptor, Tom20. We wanted to determine whether the predicted mitochondrial presequence in RECQL4 actually affected its binding and localization of RECQL4 *in vivo.* For this purpose we generated two internal deletion mutants of Flag- tagged RECQL4: Δ13-18 and Δ84. We found that while there was a reproducible 15- 20% decrease in the binding between RECQL4 and Tom20 in the Δ13-18 mutant, the binding was completely abolished in the Δ84 mutant (Figure 6E). This result indicated that additional amino acid residues in the N-terminus of RECQL4 apart from residues 13- 18 were required Tom20-RECQL4 interaction. However, unlike wild-type protein, both Δ13-18 and Δ84 RECQL4 mutants colocalized to nucleus to a high extent (Figure 6F), indicating that amino acid residues 13-18 have the capability to act as the MLS for RECQL4 *in vivo.*

**Table 1: Summary of prediction for RECQL4 for mitochondrial localization signal by the four different web based tools.**

| **S.No** | **Name of the Tool** | **Prediction** |
|---|---|---|
| **1** | **TARGET P** | P^{b} =0.82 and RC^{a} = 2 (Complete RecQL4) |
| | | P^{b} =0.82 and RC^{a} = 2 (RECQL4 1-140) |
| **2** | **PSORT** | P^{b} = 0.804 (RECQL4 1-140) |
| **3** | **MitoProf** | P^{b} =0.953 (RECQL4 1-60) |
| **4** | **iPSORT** | RecQL4 as a mitochondrial localization protein (RECQL4 1-30) |

| | | |
|---|---|---|
| **Note:** ^{a} RC is a measure of the size of the difference ('diff') between the highest and the second highest output scores. There are 5 reliability glasses, defined as follow: 1 : diff> 0.800 2 : 0.800 > diff > 0.600 3 : 0.600 > diff > 0.400 4 : 0.400 > diff > 0.200 5 : 0.200 > diff ^{b}P= Probability | | |

**Table 2: Pair potential score for the interaction between Tom20 receptor structure (both NMR and X-ray crystal structure) and the predicted mitochondrial localization sequence in RECQL4, rat ALDH pre-sequence and nuclear localization signal of DNA helicase enzyme.**

| [1] NMR structure | | | |
|---|---|---|---|
| **S. no** | **Model** | **Presequence** | **Pair potential score for the motif** |
| 1 | RECQL4 (13-19) | LQAWERAFRRQ | -9.09 |
| 2 | Rat ALDH (14-20) | GPRLSRLISYA | -10.65 |
| 3 | DNA helicase (638-644) | SKNTGAKKRKI | +1.05 |
| | | | |

| [2] X-Ray structure (A state) | | | |
|---|---|---|---|
| **S. no** | **Model** | **Presequence** | **Pair potential score for the motif** |
| 1 | RECQL4 (13-19) | LQAWERAFRAAG | -6.1 |
| 2 | Rat ALDH (14-20) | GPRISRLLSAAG | -3.45 |
| 3 | DNA helicase (638-644) | NTGAKKRKIAAG | -1.59 |
| | | | |

| [3] X-Ray structure (Y state) | | | |
|---|---|---|---|
| **S. no** | **Model** | **Presequence** | **Pair potential score for the motif** |
| 1 | RECQL4 (13-19) | LQAWERAFRYAG | -6.54 |
| 2 | Rat ALDH (14-20) | GPRLSRLLSYAG | -8.23 |
| 3 | DNA helicase (638-644) | NTGAKKRKIYAG | -0.74 |

| | | | |
|---|---|---|---|
| **Rat ALDH (14-20) is the positive control, while DNA helicase (638-644) acts as a negative ontrol** | | | |

### EXAMPLE 4:

### RECQL4 helicase activity is inhibited by p53

RECQL4 helicase assays were carried out as for Twinkle (Korhonen et al., 2003). The two DNA substrates tested were prepared using the oligoes (listed below) and subsequently PAGE purified.

Oligo 1, Oligo 2 and Oligo 3 used for making 5' single stranded tail substrate while Oligo 1 and Oligo 2 used for making splayed arm substrate.

Using splayed-arm, blunt DNA and overhang substrates, it was concluded that RECQL4 had a 3' to 5' helicase activity (Xu and Liu, 2009). (which we have confirmed, data not shown). However, on longer incubations, a 5' to 3' helicase activity for RECQL4 was detected, much like E. coli RecQ helicase (Umezu et al., 1990). We reasoned that if RECQL4 was a "bona fide" mitochondrial helicase it should also unwind similar substrate(s) as Twinkle (Korhonen et al., 2003). Purified Flag-tagged full-length RECQL4 and Twinkle (Figure 7A) could both unwind 5'-single stranded tail substrate to similar extent (Figure 7B). The unwinding activity of RECQL4 was enhanced in a protein and ATP concentration dependent manner (Figure 7C, 7D). As reported (Xu and Liu, 2009), RECQL4 mutant K508A displayed robust unwinding activity, confirming that the second ATP binding region in the N-terminus contributed to the 5' to 3' helicase activity (Figure 7E). Like for BLM and WRN (Yang et al., 2002), p53 inhibited RECQLA unwinding activity on 5' single stranded tail (Figure 7F) and splayed arm substrates (Figure 7G).

### EXAMPLE 5:

Determination of the consequence of loss of RECQL4 in cells from RTS patients: For this purpose patient fibroblasts characterized by us (Kitao et al., 1999; Lindor et al., 2000) or available from Coriell Institute of Medical Research were used. To facilitate long-term studies, the cell lines were immortalized using hTERT. Lack of RECQL4, as confirmed in immortalized RTS cells, led to the accumulation and activation of transcriptionally active nuclear p53 and thereby its downstream target genes like p21 and Bax (Figure 8). Consequently very little of the p53 was found in the mitochondrial nucleoids in the RTS fibroblasts (Figure 9A). Compared to NHF, p53 in the RTS cells was elevated to a much higher level after NCS, HU, CPT or Mito treatment. Consequently levels of p53 targets, p21 and Bax were also elevated (Figure 8). These results indicated that while p53 could not independently go to the mitochondria in absence of RECQL4, its interaction with RECQL4 in the mitochondrial nucleoids was necessary to prevent its spontaneous upregulation and conversion into a transcriptionally active form in the nucleus. We next performed long-term clonogenic assays with NHF and RTS fibroblasts. We found that the survival fraction of all the four RTS fibroblasts tested was less (compared to NHF) after DNA damage (Figure 9B). These results indicated that the lack of RECQLA coupled with abnormal nuclear accumulation of p53 sensitized the cells towards cell death induced by different types of DNA damage. Next we wanted to determine why despite enhanced p53 level, RTS cells did not undergo spontaneous apoptosis. Hence we determined the amount of intracellular cytochrome c, the mitochondrial fractions (prepared by Mitochondria Isolation Kit for Cultured Cells, Pierce) were lysed on ice according to manufacturer's protocol. Post iysis, western analysis was carried out to determine the relative levels of cytochrome c in NHF vs RTS cell lines. Decrease in level of cytochrome c, but no change in bax/bcl2 ratio indicated a possible reason for the lack of spontaneous apoptosis in RTS patient cells (Figure 9C).

### EXAMPLE 6:

Determination of the compromised mitochondrial functions in the RTS cells grown under asynchronous conditions:
For ultrastructural studies cell pellet was obtained after scrapping and centrifugation of the cultured cells followed by fixation in 4% paraformadehyde and 3 % glutaraldehyde fixative in phosphate buffer (pH 7.2) for 24 hr and subjected to routine transmission electron microscopy (TEM). Briefly the cells were post fixed in 1% osmium tetroxide for 1 hr, followed by dehydration in grades of ethyl alcohol and cleared in propylene oxide, later embedded in Araldite CY212 resin and polymerized at 60°C in oven for 48 hours. The blocks were cut in Leica EM UC6 ultramicrotome (M/S Leica Mikrosysteme, Austria). After initial screening, several ultrathin (400-500 A°) sections were collected on copper grids and stained with Uranyl acetate and Lead citrate as described by (Frasca and Parks, 1965). Sections were scanned under Tecnai 02 Spirit transmission electron microscope (FET Netherlands) and interested areas were photographed using Megaview-IJI digital CCD camera at 80 KVA.

By transmission electron microscopy (TEM) we observed that the mitochondrial cristae were distorted and the intercristal space expanded in all the RTS fibroblasts grown under asynchronous conditions (Figure 10A). The extent of the cristae space is known to affect the overall chemiosmotic function in the cell (Mannella, 2006). Since chemiosmosis is an indicator of electron transport chain (Mitchell, 1961), the TEM data indicate that the production of ATP is deficient in RTS patients not exposed to any DNA damage. Apart from ATP, mitochondria also produce the reactive oxygen species (ROS). Spontaneous ROS levels are usually low in normal cells as elevation of ROS levels can result in significant damage to cell structure (Schumacker, 2006). Indeed the level of ROS in asynchronous NHF was low as determined by the oxidation of 5-(and-6)- chloromethyl-2',7'-dichlorodihydrofluorescein diacetate, acetyl ester (CM-H2DCFDA). Interestingly all the RTS fibroblasts show a reduced level of spontaneous ROS. Citrate synthase activity, considered a biomarker for mitochondrial mass and function was decreased in all RTS fibroblasts compared to NHF (Figure 10B). Mitochondrial mass directly measured by Mitotracker Red 580 also showed the same trend (Figure 10C). Mitochondrial mass was determined by usage of a fluorescent dye Mitotracker Red 580 (200nM, Invitrogen). Live cells were incubated with of this dye for 30 min. The cells were subsequently washed, fixed and then analyzed in a Zeiss 510 Meta system with a 40x/0.95 Con objective. The fluorescence was captured initially for NHF and the acquisition parameters were kept unchanged for the capture of the fluorescence for the RTS cells. Biochemical determination of mitochondrial mass was performed by determining the citrate synthase activity in whole cell extracts using a kit (Sigma), following manufacturers' protocol. The activity was measured at 412 nm at 30°C using 10µg for each sample. Activity was expressed as nmole/min/mg of protein. Intracellular ROS in RTS cells was measured by the oxidation of CM-H2DCFDA (4µM, Invitrogen). Live cells, grown on chambered slides (Nunc), were stained with a fluorescent CM-H2DCFDA for 30 mm in phenol red free medium (without serum) at room temperature. Cells were washed, and the fluorescence was captured in a Zeiss 510 Meta system with a 20x/0.8 objective. The laser line used was Argon 458/477/488/514 nm. LSM5 software was used for image acquisition. The fluorescence was captured first for NHF and the acquisition parameters were kept unchanged for the subsequent capture of the fluorescence for the RTS cells. For the DNA damage experiments, the cells were treated with the respective drugs for 6 h. The cells were subsequently washed and the incubation continued for a further 18 h before the fluorescence readings were carried out as above. We found that the RTS cells contained abnormally high levels of ROS, 18 h after exposure to four different types of DNA damage (Figure 10D). ROS scavengers like ascorbic acid reversed this effect (Figure 10E). Expression levels of the major superoxide scavenger enzyme active in the mitochondria, superoxide dismutase 1 (SOD1) and the enzyme secreted into extracellular space, superoxide dismutase 3 (SOD3) were both decreased at protein and RNA level (Figure 11A, 11B).

### EXAMPLE 7:

### mtDNA mutations accumulate in RTS fibroblasts:

mtDNA is especially susceptible to ROS as its location is in close proximity to the sites of ROS production from the respiratory chain, apart from lack of protection by nucleosomes and limited DNA repair capabilities of mitochondria. Mutations in mtDNA have been reported in a variety of cancers (Modica-Napolitano et al., 2007). We hypothesized that high level of ROS in RTS fibroblasts could lead to accumulation of mtDNA mutations. We carried out complete mitochondrial genome sequencing of NHF and three RTS patient celllines (AG03587, B1865425K and L9552914-J). Sequencing of the entire mitochondrial genome was carried out by using the mitoSEQrTM System. This system allows identification of sequence variation in the entire human mitochondrial genome and its control regions. Overlapping region in the mitochondrial genome was amplified with specific primer pairs tailed with universal M13 sequences to generate resequencing amplicons (RSAs). The RSAs were then used as templates for sequencing using universal primers. The entire process was carried out with mitoALLTM kit (Applied Biosystem) in an Applied Biosytem 3130 Genetic Analyzer. The data was analyzed using SeqScape software v2.6. Every mutation reported was verified by duplicate analysis. Comparison with revised Cambridge Reference Sequence revealed the listed polymorphisms in NHF and RTS cells (data not shown). The three RTS fibroblasts (but not NHF) accumulated additional mutations in mtDNA (Table 3). Out of a total 44 mtDNA alterations observed in RTS patient cells, 30 were observed in L9552914-J, indicating that loss of both p53 and RECQL4 function enhanced the rate of mtDNA alterations 5-6 fold. Fifteen mutations were located in the non-coding mtDNA, the displacement loop (D-loop), known to be involved in the control of both mitochondrial replication and transcription (Clayton, 1982). In two of the three RTS patients, insertion mutations were present in a polycytidine stretch (C-tract or D310), described as a hot spot for mutations in many cancers (Sanchez-Cespedes et al., 2001) and involved in mtDNA replication and transcription (Ghivizzani et al., 1994). Out of the rest 29 alterations, 6 were non-synonymous coding region mutations (i.e. resulting in an amino-acid change in genes coding for OXPHOS enzymes), 15 were synonymous, 3 were in t-RNAs, 4 in 12/16S RNAs and one was a heteroplasmic mutation. Susceptibility of the mitochondrial coding region to DNA alterations provide an explanation regarding the perturbation in electron transport chain leading to alteration in mitochondrial potential, and generation of oxidative stress. A vast majority of mutations in RTS cells were homoplasmic in nature, supporting earlier reports of high frequency of homoplasmic mutations detected in human tumors (Mambo et al., 2003).

**Table 3: Mutations detected in the mitochondrial DNA of RECQL4 fibroblasts {reference revised Cambridge Reference Sequence (rCRS)}**

| Cells | Nucleotide change | Position of mutation | Type of mutation |
|---|---|---|---|
| AG03587 | 146T>C | D-loop | Non-coding mtDNA |
| | 195T>C | D-loop | Non-coding mtDNA |
| | 309_310insCT | D-loop | Non-coding mtDNA |
| | 310T>C | D-loop | Non-coding mtDNA |
| | 5471G>A | MT-ND2 | Synonymous mutation in NADH dehydrogenase 2 |
| | 5492T>C | MT-ND2 | Synonymous mutation in NADH dehydrogenase 2 |
| | 9145G>R | MT-ATP6 | Heteroplasmic mutation in ATP synthase 6 |
| | 12010CT | MT-ND4 | Synonymous mutation in NADH dehydrogenase 4 |
| | 14497A>G | MT-ND6 | Synonymous mutation in NADH dehydrogenase 6 |
| | | | |
| B1865425K | 152T>C | D-loop | Non-coding mtDNA |
| | 4336T>C | MT-TQ | Mutation in tRNA-glutamine |
| | 5319A>G | MT-ND2 | Non-synonymous mutation (Thr to Ala) in NADH dehydrogenase 2 |
| | 8563A>G | MT-ATP6 | Non-synonymous mutation (Thr to Ala) in ATP synthase 6 |
| | | MT-ATP8 | Synonymous mutation in ATP synthase 6 |
| | | | |
| L9552914-J | 73A>G | D-loop | Non-coding mtDNA |
| | 150C>T | D-loop | Non-coding mtDNA |
| | 310T>C | D-loop | Non-coding mtDNA |
| | 311-3312insTCC | D-loop | Non-coding mtDNA |
| | 709G>A | MT-RNR1 | Mutation in 12S RNA |
| | 1888G>A | MT-RNR2 | Mutation in 16S RNA |
| L6474163-L (cont.) | 2302A>G | MT-RNR2 | Mutation in 16S RNA |
| | 2706A>G | MT-RNR2 | Mutation in 16S RNA |
| | 4216T>C | MT-ND1 | Non-synonymous mutation (Tyr to His) in NADH dehydrogenase 1 |
| | 4917A>G | MT-ND2 | Non-synonymous mutation (Asn to Asp) in NADH dehydrogenase 2 |
| | 7028CT | MT-COI | Synonymous mutation in cytochrome c oxidase I |
| | 8697G>A | MT-ATP6 | Synonymous mutation in ATP synthase 6 |
| | 10463T>C | MT-TR | Mutation in tRNA arginine |
| | 11251A>G | MT-ND4 | Synonymous mutation in NADH dehydrogenase 4 |
| | 11719G>A | MT-ND4 | Synonymous mutation in NADH dehydrogenase 4 |
| | 11812A>G | MT-ND4 | Synonymous mutation in NADH dehydrogenase 4 |
| | 13368G>A | MT-ND5 | Synonymous mutation in NADH dehydrogenase 5 |
| | 14233A>G | MT-ND6 | Synonymous mutation in NADH dehydrogenase 6 |
| | 14766C>T | MT-CYB | Non-synonymous mutation (Thr to Ile) in cytochrome b |
| | 14905G>A | MT-CYB | Synonymous mutation in cytochrome b |
| | 15452C>A | MT-CYB | Non-synonymous mutation (Leu to Ile) in cytochrome b |
| | 15499C>T | MT-CYB | Synonymous mutation in cytochrome b |
| | 15607A>G | MT-CYB | Synonymous mutation in cytochrome b |
| | 15928G>A | MT-TT | Mutation in tRNA threonine |
| | 16114C>T | D-loop | Non-coding mtDNA |
| | 16126T>C | D-loop | Non-coding mtDNA |
| | 16153G>A | D-loop | Non-coding mtDNA |
| | 16192C>T | D-loop | Non-coding mtDNA |
| | 16294C>T | D-loop | Non-coding mtDNA |
| | 16519T>C | D-loop | Non-coding mtDNA |

### EXAMPLE 8:

Loss of both p53 and RECQLA led to increased mtDNA mutations and enhanced metastatic potential of RTS fibroblasts.

Next we wanted to determine whether enhanced mtDNA mutations in RTS patients can be correlated to the functional status of RECQL4 and p53 in RTS patient cells. Hence we studied the relative metastatic potential of NHF and RTS cells using BD BioCoat™ Matrigel™ Invasion Chamber. 106 cells/cm2 was seeded in 24-well chamber and incubated for 22 h at 370C. The non-invading cells were removed by scrubbing, the cells which have migrated to the lower surface of the membrane were fixed by 100% methanol, stained with 1% Toluidine blue, photographed at 63X in Upright Axioimager M1 motorized Epifluorescence microscope equipped with a high resolution AxioCam MRm Rev. 2 camera. The experiment was done in triplicate for three times and data represented is combined from all datasets. HCT116, used as a positive control, demonstrated robust invasion through the basement membrane. While NHF showed negligible invasive capability, RTS fibroblasts invaded the basement membrane, but to varied extent (**Figure 11C**). The extent of invasion of L9552914-J was even greater than HCT116 and was about 10-fold more than AG03587 and B1865425K. The relative invasive capabilities of the tested cells mirrored the number of mtDNA mutations, thereby indicating that mitochondrial deregulation in RTS patients probably plays a very important role during cancer development

### Advantages of the inventions

This work provides an insight into the potential diverse mitochondrial functions of RECQL4. Further, it unfolds the functions of RECQL4 which may be involved in increasing fidelity of the mitochondrial replication process and/or regulating the diverse processes that control the Reactive Oxygen Species (ROS) level.

These findings indicate that RECQL4/RECQL4 variant-p53 adduct can be a target for development of vaccines, and as a first generation vaccine candidate, GFP-tagged RECQL4 can be cloned into lentivirus vector, transduced into RTS patient cells and constitutively expressed. The "rescued" patient cells can be scored for the mitochondrial functions.

For a second generation vaccine candidate, an exogenous mitochondrial import leader peptide can be attached to the GFP-tagged RECQL4 so that the mitochondrial localization of the helicase is faster and complete. This modified full-length RECQL4 with the exogenous mitochondrial leader peptide can be constitutively expressed using the lentivirus system.

For a third generation vaccine candidate, modifications of the wildtype GFP-tagged RECQL4 can be done that a minimum amount of the helicase is expressed for the maximum efficacy. Recombinant GFP-RECQL4 construct can be generated which will contain the mitochondrial localization signal (either its own or an exogenous peptide), the minimum domain which would allow RECQL4 to bind to p53 and the helicase domain through which RECQL4 probably affects mitochondrial replication. These GFP-tagged constructs can be cloned into the lentivirus expression system, expressed into patient cells and scored for mitochondrial functions.

## Claims

1. A method of identification of functional RECQL4/RECQL4 variant -p53 complex and of mitochondrial DNA mutations in Rothmund-Thomson Syndrome (RTS), wherein the said method comprises the steps of:
a) interaction of p53 and RECQL4/RECQL4 variant
b) import of RECQL4/RECQL4 variant and p53 complex through mitochondrial Tom 40 receptor complex.
c) import of RECQL4/RECQL4 variant and p53 complex into mitochondria through Tom 40 receptor complex via the mitochondrial localization signal (MLS) of RECQL4.
d) the localization of the p53-RECQL4/RECQL4 variant complex in the mitochondrial nucleoids and;
measurement of reactive oxygen species (ROS), and identification of mitochondrial DNA mutations in Rothmund-Thomson Syndrome.

2. A method as claimed in claim 1, wherein the interaction of RECQL4 and p53 is through the nuclear localization signal of both RECQL4 and p53.

3. A method as claimed in claim 1, wherein the MLS of RECQL4 is located at the N-terminal region of RECQL4.

4. A method as claimed in claim 3, wherein MLS of RECQL4 is located between the 13th and 18th peptide of the N-terminal region of RECQL4.

5. A method as claimed in claim 1, wherein mitochondrial mutations identifies RTS.

## Patentansprüche

1. Verfahren zur Identifizierung eines Komplexes aus funktionaler RECQL4/RECQL4-Variante und p53 und von mitochondrialen DNS-Mutationen beim Rothmund-Thomson-Syndrom (RTS), worin das Verfahren die Schritte umfasst:
a) Wechselwirkung von p53 und RECQL4/RECQL4-Variante;
b) Import des Komplexes aus RECQL4/RECQL4-Variante und p53 durch den mitochondrialen TOM-40-Rezeptorkomplex;
c) Import des Komplexes aus RECQL4/RECQL4-Variante und p53 in Mitochondrien durch den TOM-40-Rezeptorkomplex mittels des mitochondrialen Lokalisierungssignals (MLS) von RECQL4;
d) Lokalisierung des Komplexes aus p53 und RECQL4/RECQL4-Variante in den mitochondrialen Nukleoide; und
Messung der reaktiven Sauerstoffspezies (ROS) und Identifizierung von mitochondrialen DNS-Mutationen beim Rothmund-Thomson-Syndrom.

2. Verfahren nach Anspruch 1, worin die Wechselwirkung von RECQL4 und p53 durch die Kernlokalisierungssignale sowohl von RECQL4 als auch von p53 erfolgt.

3. Verfahren nach Anspruch 1, worin das MLS von RECQL4 in der N-Terminalregion von RECQL4 lokalisiert ist.

4. Verfahren nach Anspruch 3, worin das MLS von RECQL4 zwischen dem 13. und dem 18. Peptid der N-Terminalregion von RECQL4 lokalisiert ist.

5. Verfahren nach Anspruch 1, worin mitochondriale Mutationen RTS identifizieren.

## Revendications

1. Procédé d'identification d'un complexe variant RECQL4/RECQL4-p53 fonctionnel et de mutations de l'ADN mitochondrial dans le syndrome de Rothmund-Thomson (SRT), lequel procédé comprend les étapes consistant en:
a) l'interaction de p53 et du variant RECQL4/RECQL4
b) l'importation du complexe variant RECQL4/RECQL4 et p53 à travers le complexe mitochondrial Tom 40 récepteur.
c) l'importation du complexe variant RECQL4/RECQL4 et p53 dans les mitochondries à travers le complexe Tom 40 récepteur par l'intermédiaire du signal de localisation mitochondriale (SLM) de RECQL4.
d) la localisation du complexe variant p-53-RECQL4/RECQL4 dans les nucléoïdes mitochondriaux et;
la mesure des espèces réactives de l'oxygène (ERO) et l'identification des mutations de l'ADN mitochondrial dans le syndrome de Rothmund-Thomson.

2. Procédé selon la revendication 1, dans lequel l'interaction de RECQL4 et de p53 est par l'intermédiaire du signal de localisation nucléaire tant de RECQL4 que de p53.

3. Procédé selon la revendication 1, dans lequel le SLM de RECQL4 se situe à la région N-terminale de RECQL4.

4. Procédé selon la revendication 3, dans lequel le SLM de RECQL4 se situe entre le 13^{e} et le 18^{e} peptide de la région N-terminale de RECQL4.

5. Procédé selon la revendication 1, dans lequel des mutations mitochondriales identifient un SRT.
